# FASCICULE DE BREVET EUROPEEN

(11) **EP 1 584 321 B1**
(45) Date de publication et mention de la délivrance du brevet: **15.10.2008**
(21) Numéro de dépôt: 05290628.6
(22) Date de dépôt: 22.03.2005
(51) Int. Cl.: A61Q 1/02, A61K 8/90, A61K 8/87

(54) **Composition cosmétique anhydre comprenant une huile, un gélifiant polymérique et une poudre de polyuréthane**
Wasserfreie kosmetische Zusammensetzung, die ein Öl, ein polymeres Gelbildungsmittel und ein Polyurethanpulver enthält
Anhydrous cosmetic composition comprising an oil, a polymeric gelling agent and a polyurethane powder

(30) Priorité: 06.04.2004 FR 0450696; 20.04.2004 FR 0404186
(43) Date de publication de la demande: 12.10.2005
(73) Titulaire: L'ORÉAL, 75008 Paris (FR)
(72) Inventeur: Themens, Agnès, 92340 Bourg La Reine (FR); Leuridan, Maitena, 75005 Paris (FR)
(74) Mandataire: Boulard, Denis

(56) Documents cités:
- EP-A- 1 002 528
- FR-A- 2 840 204
- FR-A- 2 843 020

## Description

La présente invention a pour objet une composition cosmétique anhydre de maquillage ou de soin de la peau. L'invention a également pour objet un procédé de maquillage ou de soin de la peau d'être humain comprenant l'application de la composition sur la peau.

La composition de maquillage de la peau peut être un fond de teint, un fard à paupières, un fard à joue, un produit anticernes, un produit de maquillage du corps. Plus spécialement, l'invention porte sur une composition de fond de teint.

La composition de soin peut notamment être une base de maquillage, un produit matifiant pour la peau, un produit de soin de la peau.

La composition est de préférence une composition de maquillage de la peau.

Les compositions de fond de teint sont couramment employées pour apporter une couleur esthétique à la peau, notamment au visage, mais également pour camoufler les imperfections de la peau telles que les rougeurs, les taches.

Ces compositions présentent des textures variées allant du fluide au solide et contiennent généralement des huiles et des matières colorantes pulvérulentes. Une des difficultés rencontrées par les utilisatrices est de pouvoir bien étaler uniformément le fond de teint sur toute la surface du visage de manière à répartir uniformément le produit. Les compositions de textures épaisses ou solides sont difficiles à étaler en raison de leurs viscosités élevées. Les compositions de textures fluides ne sont pas toujours appropriées pour obtenir un maquillage uniforme, ne laissant pas de marques visibles sur la peau, notamment en raison de leur mauvais étalement sur toute la surface du visage à maquiller. Par ailleurs, la présence de matières pulvérulentes peut engendrer un effet de dessèchement au maquillage, conduisant à une sensation de tiraillement, rendant ainsi le maquillage inconfortable à porter pendant la journée.

Les consommatrices sont toujours à la recherche de produits novateurs dans les effets de texture. En particulier, il est recherché des produits présentant des textures différentes avant et après application du produit de maquillage ou de soin de la peau.

Le but de la présente invention est donc de disposer d'une composition de maquillage ou de soin ayant l'aspect d'une crème onctueuse et se transformant en un fini poudré après son application sur la peau. Un autre but de l'invention est de disposer d'une composition présentant une sensation de douceur lors de son application sur la peau. Un autre but de l'invention est de disposer d'une composition présentant de bonnes propriétés de tenue, en particulier de tenue vis-à-vis du sébum ou de la sueur, notamment au bout de douze heures après l'application de la composition.

Les inventeurs ont découvert qu'une telle composition est obtenue en utilisant une huile, un gélifiant polymérique particulier et une poudre de polyuréthane.

De façon plus précise, l'invention a pour objet une composition anhydre de maquillage ou de soin de la peau comprenant une phase huileuse, un polymère gélifiant amorphe formé par polymérisation d'une oléfine, et une poudre de polyuréthane.

L'invention a également pour objet un procédé cosmétique de maquillage ou de soin non thérapeutique de la peau comprenant l'application sur la peau d'une composition telle que définie précédemment.

La composition selon l'invention présente une texture crème onctueuse et fondante lors de son application sur la peau. La composition s'étale facilement sur la peau et permet d'obtenir un maquillage uniforme de la peau sans laisser de traces visibles. De plus, après l'application sur la peau, le maquillage ou le dépôt obtenu présente un fini poudré, velouté et est confortable à porter, sans effet de dessèchement, de tiraillement ; la peau maquillée ou traitée présente une douceur agréable. De plus, le maquillage obtenu possède de bonnes propriétés de tenue, notamment de tenue au sébum ou à la sueur : après une durée de 12 heures après l'application de la composition, le maquillage reste toujours sur la peau, est homogène.

L'invention a donc également pour objet l'utilisation d'une composition telle que définie précédemment pour obtenir un maquillage ayant une bonne tenue, notamment après 12 heures, et/ou une peau maquillée douce et/ou un maquillage confortable et/ou un maquillage ayant un aspect poudré et/ou un maquillage homogène.

On entend par composition anhydre une composition contenant moins de 2 % en poids d'eau, voire moins de 0,5 % d'eau, et notamment exempte d'eau, l'eau n'étant pas ajoutée lors de la préparation de la composition mais correspondant à l'eau résiduelle apportée par les ingrédients mélangés.

Le gélifiant polymérique présent dans la composition selon l'invention est un polymère amorphe formé par polymérisation d'une oléfine. L'oléfine peut être notamment un monomère à insaturation éthylénique élastomérique. Comme exemple d'oléfine, on peut citer les monomères de carbure éthylénique, ayant notamment une ou deux insaturations éthyléniques, ayant de 2 à 5 atomes de carbone tels que l'éthylène, le propylène, le butadiène, l'isoprène.

Le gélifiant polymérique est capable d'épaissir ou de gélifier la phase organique de la composition. Par polymère amorphe, on entend un polymère qui n'a pas de forme cristalline. Le gélifiant polymérique est également filmogène, c'est-à-dire qu'il est capable de former un film lors de son application sur la peau.

Le gélifiant polymérique peut être notamment un copolymère dibloc, tribloc, multibloc, radial, étoile, ou leurs mélanges.

De tels gélifiants polymériques sont décrits dans la demande US-A-2002/005562 et dans le brevet US-A-5 221 534
Avantageusement, le gélifiant polymérique est un copolymère bloc amorphe de styrène et d'oléfine.
Le gélifiant polymérique est de préférence hydrogéné pour réduire les insaturations éthyléniques résiduelles après la polymérisation des monomères.

En particulier, le gélifiant polymérique est un copolymère, éventuellement hydrogéné, à blocs styrène et à blocs éthylène/alkylène en C₃-C₄.

Comme copolymère dibloc, de préférence hydrogéné, on peut citer les copolymères de styrène-éthylène/propylène, les copolymères de styrène-éthylène/butadiène. Des polymères diblocs sont notamment vendus sous la dénomination Kraton® G1701 E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

On peut également utiliser un mélange de copolymère hydrogéné tribloc styrène-butylène/éthylène-styrène et de polymère étoile hydrogéné éthylène-propylène-styrène, un tel mélange étant notamment dans l'isododécane. De tels mélanges sont par exemple vendus par la société PENRECO sous les dénominations commerciales VERSAGEL® M5960 et VERSAGEL® M5670.

Avantageusement, on utilise comme gélifiant polymérique un copolymère dibloc tel que ceux décrits précédemment, en particulier un copolymère dibloc de styrène-éthylène/propylène.

Le gélifiant polymérique peut être présent en une teneur allant de 0,1 % à 10 % en poids, par rapport au poids total de la composition, de préférence allant de 0,5 % à 5 % en poids, et préférentiellement allant de 1 % à 3 % en poids.

La composition selon l'invention contient une poudre de polyuréthanequi n'est pas filmogène, c'est-à-dire qu'elle ne forme pas un film continu lorsqu'elle est déposée sur un support tel que la peau.

Avantageusement, la poudre de polyuréthane est une poudre de copolymère de diisocyanate d'hexaméthylène et de triméthylol hexyl lactone. Une telle poudre de polyuréthane est notamment vendue sous les dénominations « PLASTIC POWDER D-400 », « PLASTIC POWDER D-800 » par la société TOSHIKI.

Comme autre poudre de polyuréthane, on peut utiliser celle vendue sous la dénomination « PLASTIC POWDER CS-400 » par la société TOSHIKI.

La poudre de polyuréthane peut être présente en une teneur allant de 0,5 % à 30 % en poids, par rapport au poids total de la composition, et de préférence allant de 1 % à 15 % en poids, et préférentiellement allant de 5 % à 15 % en poids.

Selon un mode de réalisation de l'invention, la composition peut comprendre des particules de polyméthacrylate de méthyle. En particulier, ces particules ne sont pas filmogènes , c'est-à-dire qu'elles ne forment pas un film continu lorsqu'elle sont déposées sur un support tel que la peau.

Les poudres de polyméthacrylate de méthyle se présentent généralement sous la forme de particules sphériques creuses ou pleines de couleur blanche dont la taille moyenne en nombre est généralement à l'échelle du micromètre, en particulier varie de 3 à 15 microns et généralement varie de 3 à 10 microns. Par « taille moyenne en nombre », on désigne la dimension donnée par la distribution granulométrique statistique à la moitié de la population, dite D50.

Il est également possible de caractériser ces particules de polyméthacrylate de méthyle par leur densité, celle-ci étant susceptible de varier notamment en fonction de la taille de la cavité sphérique desdites particules.
Dans le cadre de la présente invention, cette densité est appréciée selon le protocole suivant, dit de la densité tassée :
On verse m=40 g de poudre dans une éprouvette graduée ; puis on place l'éprouvette sur l'appareil STAV 2003 de chez STAMPF VOLUMETER ; l'éprouvette est ensuite soumise à 1500 tassements ; puis on mesure directement sur l'éprouvette le volume final Vf de poudre tassée. La densité tassée est déterminée par le rapport m/Vf, en l'occurrence 40/Vf (Vf étant exprimé en cm³ et m en g).
En particulier, la densité des particules de polyméthacrylate de méthyle utilisables selon l'invention peut varier de 0,3 à 1,5 , notamment de 0,5 à 1,5 , et plus particulièrement de 1 à 1,5.

A titre représentatif et non limitatif de polyméthacrylate de méthyle convenant à l'invention, on peut notamment citer les particules de polyméthyméthacrylate commercialisées par la société MATSUMOTO YUSHI CO sous la dénomination « Mieropearl M100 », par la société LCW sous la dénomination « Covabead LH 85 » et celles commercialisées par la société NIHON JUNYAKU sous la dénomination « JURYMER MB1 ».

Les particules de polyméthacrylate de méthyle peuvent être présentes en une teneur allant de 0,5 % à 30 % en poids, par rapport au poids total de la composition, de préférence allant de 0,5 % à 25 % en poids, préférentiellement allant de 1 % à 20 % en poids, et plus préférentiellement allant de 5 % à 15 % en poids.

La poudre de polyméthacrylate de méthyle permet notamment d'obtenir un produit de maquillage ou de soin matifiant.

La phase huileuse de la composition selon l'invention comprend au moins une huile pouvant être choisie parmi les huiles volatiles, les huiles non volatiles , et leurs mélanges. Avantageusement, la composition comprend au moins une huile volatile et au moins une huile non volatile.

La composition selon l'invention peut comprendre au moins une huile volatile.

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

L'huile volatile peut être choisie parmi les huiles volatiles hydrocarbonées, les huiles volatiles siliconées, les huiles volatiles fluorées, et leurs mélanges.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permethyls®.

Comme huiles volatiles, on peut aussi utiliser les silicones volatiles, comme par exemple les huiles de silicones linéaires ou cycliques volatiles, notamment celles ayant une viscosité ≤ 5 centistokes (5 x 10⁻⁶ m²/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile fluorée n'a généralement pas de point éclair.
Comme huile volatile fluorée, on peut citer le nonafluoroéthoxybutane, le nonafluorométhoxybutane, le décafluoropentane, le tétradécafluorohexane, le dodécafluoropentane, et leurs mélanges.

Avantageusement, la composition comprend au moins une huile volatile hydrocarbonée, et notamment un mélange d'isododécane et d'isohexadécane.

L'huile volatile peut être présente dans la composition selon l'invention en une teneur allant de 5 % à 60 % en poids, par rapport au poids total de la composition, de préférence allant de 10 % à 55 % en poids, préférentiellement allant de 20 % à 50 % en poids, et plus préférentiellement allant de 30 % à 50 % en poids.

La composition selon l'invention peut comprendre au moins une huile non volatile.

Par "huile non volatile", on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides capryliquelcaprique comme ceux vendus par la société Stéarineries Dubois ou ceux vendus sous les dénominations Miglyol 810®, 812® et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam ®, le squalane , les huiles de paraffine, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

Avantageusement, l'huile non volatile peut être choisie parmi les esters en C₁₂-C₃₆ tels que ceux décrits précédemment.

L'huile non volatile peut être présente en une teneur allant de 0,5 % à 60 % en poids, par rapport au poids total de la composition, de préférence allant de 1 % à 50 % en poids, de préférence allant de 5 % à 40 % en poids, préférentiellement allant de 5 % à 30 % en poids, et plus préférentiellement allant de 10 % à 20 % en poids.

De préférence, lorsque la composition selon l'invention comprend des particules de polyméthacrylate de méthyle, les teneurs en huile non volatile et en particules de polyméthacrylate de méthyle sont ajustées de telle sorte que la teneur en huile non volatile soit supérieure à la teneur en particules de polyméthacrylate de méthyle. En particulier, ces teneurs sont telles que le rapport pondéral huile non volatile / particules de polyméthacrylate de méthyle peut être supérieur ou égal à 1, notamment allant de 1 à 35, de préférence allant de 1 à 25, et préférentiellement allant de 1 à 15 ; de préférence, le rapport pondéral huile non volatile / particules de polyméthacrylate de méthyle peut être supérieur ou égal à 1,5 , notamment allant de 1, 5 à 35, de préférence allant de 1,5 à 25, et préférentiellement allant de 1,5 à 15.

La composition selon l'invention peut comprendre au moins une matière colorante, notamment choisie parmi les pigments, les nacres, les colorants liposolubles, et leurs mélanges.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.
Par « colorants », il faut comprendre des composés généralement organiques solubles dans les corps gras comme les huiles.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.

Les matières colorantes peuvent être présentes en une teneur allant de 0,1 % à 30 % en poids, par rapport au poids total de la composition, de préférence allant de 0,1 % à 20 % en poids, de préférence allant de 0,5 % à 15 % en poids, préférentiellement allant de 1 % à 15 % en poids, et plus préférentiellement allant de 5 % à 15 % en poids.

La composition selon l'invention peut comprendre également au moins une charge additionnelle, différente de la poudre de polyuréthane et des particules de polyméthacrylate de méthyle décrites précédemment.

Par charges, il faut comprendre des particules de toute forme, incolores ou blanches, minérales ou de synthèse, insolubles dans le milieu de la composition quelle que soit la température à laquelle la composition est fabriquée.

Les charges additionnelles peuvent être minérales ou organiques de toute forme, plaquettaires, sphériques ou oblongues, quelle que soit la forme cristallographique (par exemple feuillet, cubique, hexagonale, orthorombique, etc) . On peut citer le talc, le mica, la silice, le kaolin, les poudres de polyamide (Nylon®) , les poudres de poly-β-alanine, les poudres de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les poudres de résine de silicone, notamment les poudres de silsesquioxane (poudres de résine de silicone notamment décrites dans le brevet EP 293795 ; Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; et leurs mélanges.

Les charges additionnelles peuvent être présentes dans la composition en une teneur allant de 0,1 % à 35 % en poids, par rapport au poids total de la composition, de préférence allant de 0,5 % à 30 % en poids, et préférentiellement allant de 1 % à 25 % en poids.

Avantageusement, les charges additionnelles sont choisies parmi les poudres de silsesquioxane , le talc, les poudres de polyamide (Nylon®), et leurs mélanges.

La composition peut également comprendre une poudre de silsesquioxane en une teneur allant de 0,5 % à 30 % en poids, par rapport au poids total de la composition, et de préférence allant de 1 % à 15 % en poids, et préférentiellement allant de 1 % à 10 % en poids.

La composition peut aussi comprendre du talc en une teneur allant de 0,1 % à 30 % en poids, par rapport au poids total de la composition, et de préférence allant de 1 % à 15 % en poids, et préférentiellement allant de 1 % à 10 % en poids.

La composition peut comprendre de la poudre de polyamide en une teneur allant de 0,5 % à 30 % en poids, par rapport au poids total de la composition, et de préférence allant de 1 % à 15 % en poids, et préférentiellement allant de 1 % à 10 % en poids.

Avantageusement, la composition selon l'invention peut comprendre une teneur totale en matières pulvérulentes allant de 20 % à 50 % en poids, par rapport au poids total de la composition, de préférence allant de 25 % à 45 % en poids, et préférentiellement allant de 30 % à 40 % en poids.

La composition peut également comprendre au moins un épaississant minéral de milieu huileux tel qu'une argile organophile, les silices pyrogénées.

Les argiles organophiles sont des argiles modifiées par des composés chimiques rendant l'argile apte à gonfler dans les milieux huileux.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.
Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques comme la peau.

L'argile organophile peut être choisie parmi la montmorrilonite, la bentonite, l'hectorite, l'attapulgite, la sépiolite , et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

L'épaississant minéral de milieu huileux peut être présent dans la composition en une teneur allant de 0,5 % à 5 % en poids, par rapport au poids total de la composition, de préférence en une teneur allant de 1 % à 4 % en poids, et préférentiellement allant de 2 % à 4 % en poids.

La composition peut contenir d'autres ingrédients cosmétiques usuels pouvant être choisis notamment parmi les antioxydants, les parfums, les conservateurs, les neutralisants, les tensioactifs, les cires, les filtres solaires, les vitamines, les hydratants, les composés auto-bronzants, les actifs antirides.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Avantageusement, la composition se présente sous la forme d'un gel non fluide déformable : la composition ne s'écoule pas sous son propre poids à 25 °C en moins de 5 minutes et elle est déformable par simple écrasement lors de la prise aux doigts de la composition (contrairement à une composition solide qui ne se déforme pas lors de son simple contact avec les doigts).

Selon un mode particulier de réalisation de l'invention, la composition est exempte de particules de polymères dispersées dans la phase huileuse de la composition et stabilisées en surface par un polymère stabilisant. De telles dispersions de polymère stabilisées sont notamment décrites dans la demande EP-A-749747. Par "exempte de", on entend que la composition contient moins de 3 % en poids, par rapport au poids total de la composition, de particules de polymères dispersées et stabilisées en surface avec un polymère stabilisant, voire ne contient pas de telles particules. En particulier, ces dispersions stables ont la particularité de rester sous forme de dispersion lorsqu'elles sont diluées à 50 % en volume avec leur milieu de dispersion.

L'invention est illustrée plus en détails par l'exemple décrit ci-après.

### Exemple 1 :

On a préparé un fond de teint ayant la composition suivante :

| | |
|---|---|
| Isononyle isononanoate | 15,7 g |
| Isododécane | 11,75 g |
| Isohexadécane | 14,1 g |
| Mono-isostéarate de sorbitane | 1,5 g |
| Copolymère styrène/éthylène-propylène vendu sous la dénomination KRATON G 1701 E par la société Kraton Polymers | 2,5 g |
| Gel de Quaternium-18 Hectorite vendu sous la dénomination "Bentone gel SS 71 V" par la société ELEMENTIS | 17,65 g |
| Poudre de polyuréthane vendue sous la dénomination "PLASTIC POWDER D-400" par la société TOSHIKI | 8 g |
| Poudre de polyméthacrylate de méthyle | 9 g |
| Poudre de polyméthylsilsesquioxane vendue sous la dénomination "TOSPEARL 145-A" par la société GE TOSHIBA SILICONES | 1,25 g |
| Talc | 5,5 g |
| Silice pyrogénée vendue sous la dénomination "AEROSIL 200" par la société DEGUSSA | 1,8 g |
| Poudre de nylon | 2,75 g |
| Pigments | 8 g |
| Conservateurs | 0,5 g |

Ce fond de teint présente une texture crème onctueuse, fondante lors de son application sur la peau ; il s'étale facilement sur la peau, sans faire de bruit crissant, et permet d'obtenir un maquillage de la peau homogène, d'aspect mat, ayant un fini poudré, et laisse la peau très douce. 12 heures après l'application, le maquillage présente ainsi qu'une bonne tenue au sébum et à la sueur.

## Revendications

1. Composition anhydre de maquillage ou de soin de la peau contenant moins de 2 % en poids d'eau et comprenant une phase huileuse, un polymère gélifiant amorphe formé par polymérisation d'une oléfine et au moins une poudre de polyuréthane non filmogène.

2. Composition selon la revendication précédente, **caractérisée par le fait que** le gélifiant polymérique est un copolymère bloc amorphe de styrène et d'oléfine.

3. Composition selon l'une quelconque des revendications précédentes, **caractérisée par le fait que** le gélifiant polymérique est formé par polymérisation de monomères de carbure éthylénique, ayant notamment une ou deux insaturations éthyléniques, ayant de 2 à 5 atomes de carbone.

4. Composition selon l'une quelconque des revendications précédentes, **caractérisée par le fait que** le gélifiant polymérique est formé par polymérisation d'oléfine choisie parmi l'éthylène, le propylène, le butadiène, l'isoprène.

5. Composition selon l'une quelconque des revendications précédentes, **caractérisée par le fait que** le gélifiant polymérique est choisi parmi les copolymères dibloc, tribloc, multibloc, radial, étoile, ou leurs mélanges.

6. Composition selon l'une quelconque des revendications précédentes, **caractérisée par le fait que** le gélifiant polymérique est choisi parmi les copolymères, tribloc, multibloc, radial, étoile, ou leurs mélanges.

7. Composition selon l'une quelconque des revendications précédentes, **caractérisée par le fait que** le gélifiant polymérique est un copolymère, éventuellement hydrogéné, à blocs styrène et à blocs éthylène/alkylène en C₃-C₄.

8. Composition selon l'une quelconque des revendications 1 à 5 et 7, **caractérisée par le fait que** le gélifiant polymérique est un copolymère dibloc, de préférence hydrogéné, choisi parmi les copolymères de styrène-éthylène/propylène, de styrène-éthylène/butadiène, et de préférence un copolymère dibloc de styrène-éthylène/propylène.

9. Composition selon l'une quelconque des revendications 1 à 7, **caractérisée par le fait que** le gélifiant polymèrique est un copolymère tribloc, de préférence hydrogéné, choisi parmi les copolymères de styrène-éthylène/propylène-styrène, les copolymères de styrène-éthylène/butadiène-styrène, les copolymères de styrène-isoprène-styrène, les copolymères de styrène-butadiène-styrène.

10. Composition selon l'une quelconque des revendications 1 à 7, **caractérisée par le fait que** le gélifiant polymérique est un mélange de copolymère hydrogéné tribloc styrène-butylène/éthylène-styrène et de polymère étoile hydrogéné styrène-propylène-styrène.

11. Composition selon l'une quelconque des revendications précédentes, **caractérisée par le fait que** le gélifiant polymérique est présent en une teneur allant de 0,1 % à 10 % en poids, par rapport au poids total de la composition, de préférence allant de 0,5 % à 5 % en poids, et préférentiellement allant de 1 % à 3 % en poids.

12. Composition selon l'une quelconque des revendications précédentes, **caractérisée par le fait que** la poudre de polyuréthane est une poudre de copolymère de diisocyanate d'hexaméthylène et de triméthylol hexyl lactone.

13. Composition selon l'une quelconque des revendications précédentes, **caractérisée par le fait qu'**elle comprend une poudre de polyuréthane en une teneur allant de 0,5 % à 30 % en poids, par rapport au poids total de la composition, et de préférence allant de 1 % à 15 % en poids, et préférentiellement allant de 5 % à 15 % en poids.

14. Composition selon l'une quelconque des revendications précédentes, **caractérisée par le fait qu'**elle comprend des particules de polyméthacrylate de méthyle.

15. Composition selon l'une quelconque des revendications précédentes, **caractérisée par le fait que** les particules de polyméthacrylate de méthyle sont présentes en une teneur allant de 0,5 % à 30 % en poids, par rapport au poids total de la composition, de préférence allant de 0,5 % à 25 % en poids, préférentiellement allant de 1 % à 20 % en poids, et plus préférentiellement allant de 5 % à 15 % en poids.

16. Composition selon l'une quelconque des revendications précédentes, **caractérisée par le fait qu'**elle comprend une huile volatile.

17. Composition selon l'une quelconque des revendications précédentes, **caractérisée par le fait qu'**elle comprend une huile volatile hydrocarbonée.

18. Composition selon l'une quelconque des revendications précédentes, **caractérisée par le fait qu'**elle comprend une huile volatile hydrocarbonée choisie parmi les huiles volatiles hydrocarbonées ayant de 8 à 16 atomes de carbone et leurs mélanges, et notamment les alcanes ramifiés en C₈-C₁₆.

19. Composition selon l'une quelconque des revendications précédentes, **caractérisée par le fait qu'**elle comprend une huile volatile hydrocarbonée choisie parmi l'isododécane, l'isodécane, l'isohexadécane, et leurs mélanges.

20. Composition selon l'une quelconque des revendications précédentes, **caractérisée par le fait qu'**elle comprend un mélange d'isododécane et d'isohexadécane.

21. Composition selon l'une quelconque des revendications précédentes, **caractérisée par le fait qu'**elle comprend une huile volatile siliconée.

22. Composition selon la revendication précédente, **caractérisée par le fait que** l'huile volatile siliconée est choisie parmi l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

23. Composition selon l'une quelconque des revendications 16 à 22, **caractérisée par le fait que** l'huile volatile est présente en une teneur allant de 5 % à 60 % en poids, par rapport au poids total de la composition, de préférence allant de 10 % à 55 % en poids, préférentiellement allant de 20 % à 50 % en poids, et plus préférentiellement allant de 30 % à 50 % en poids.

24. Composition selon l'une quelconque des revendications précédentes, **caractérisée par le fait qu'**elle comprend une huile non volatile.

25. Composition selon l'une quelconque des revendication précédentes, **caractérisée par le fait qu'**elle comprend une huile non volatile choisie parmi les huiles hydrocarbonées non volatiles, les huiles siliconées non volatiles, et leurs mélanges.

26. Composition selon l'une quelconque des revendications précédentes, **caractérisée par le fait qu'**elle comprend une huile non volatile choisie parmi les esters en C₁₂-C₃₆.

27. Composition selon l'une quelconque des revendications 24 à 26, **caractérisée par le fait que** l'huile non volatile est présente en une teneur allant de 0,5 % à 60 % en poids, par rapport au poids total de la composition, de préférence allant de 1 % à 50 % en poids, de préférence allant de 5 % à 40 % en poids, préférentiellement allant de 5 % à 30 % en poids, et plus préférentiellement allant de 10 % à 20 % en poids.

28. Composition selon l'une quelconque des revendications 24 à 27, combinée avec l'une des revendications 13 et 14, **caractérisée par le fait que** l'huile non volatile est présente en une teneur supérieure à la teneur en particules de polyméthacrylate de méthyle.

29. Composition selon l'une quelconque des revendications 24 à 28, combinée avec l'une des revendications 13 et 14, **caractérisée par le fait que** l'huile non volatile et les particules de polyméthacrylate de méthyle sont présentes en une teneur telle que le rapport pondéral huile non volatile / particules de polyméthacrylate de méthyle est supérieur ou égal à 1, notamment allant de 1 à 35, de préférence allant de 1 à 25, et préférentiellement allant de 1 à 15.

30. Composition selon l'une quelconque des revendications 24 à 28, combinée avec l'une des revendications 13 et 14, **caractérisée par le fait que** l'huile non volatile et les particules de polyméthacrylate de méthyle sont présentes en une teneur telle que le rapport pondéral huile non volatile / particules de polyméthacrylate de méthyle est supérieur ou égal à 1,5 , notamment allant de 1,5 à 35, de préférence allant de 1,5 à 25, et préférentiellement allant de 1,5 à 15.

31. Composition selon l'une quelconque des revendications précédentes, **caractérisée par le fait qu'**elle comprend au moins une matière colorante.

32. Composition selon la revendication précédente, **caractérisée par le fait que** la matière colorante est choisie parmi les pigments, les nacres, les colorants liposolubles, et leurs mélanges.

33. Composition selon l'une des revendications 31 ou 32, **caractérisée par le fait que** la matière colorante est présente en une teneur allant de 0,1 % à 30 % en poids, par rapport au poids total de la composition, de préférence allant de 0,1 % à 20 % en poids, de préférence allant de 0,5 % à 15 % en poids, préférentiellement allant de 1 % à 15 % en poids, et plus préférentiellement allant de 5 % à 15 % en poids.

34. Composition selon l'une quelconque des revendications précédentes, **caractérisée par le fait qu'**elle comprend une charge additionnelle différente de la poudre de polyuréthane et des particules de polyméthacrylate de méthyle.

35. Composition selon la revendication précédente, **caractérisée par le fait que** la charge additionnelle est choisie parmi le talc, le mica, la silice, le kaolin, les poudres de polyamide, les poudres de poly-β-alanine, les poudres de polyéthylène, les poudres de polymères de tétrafluoroéthylène, la lauroyl-lysine, l'amidon, le nitrure de bore, les microsphères creuses de chlorure de polyvinylidène/acrylonitrile, les microsphères creuses de copolymères d'acide acrylique, les poudres de résine de silicone, les particules de polyorganosiloxanes élastomères, le carbonate de calcium précipité, le carbonate de magnésium, l'hydro-carbonate de magnésium, l'hydroxyapatite, les microcapsules de verre ou de céramique, les savons métalliques dérivés d'acides organiques carboxyliques ayant de 8 à 22 atomes de carbone, et leurs mélanges.

36. Composition selon la revendication précédente, **caractérisée par le fait que** la charge additionnelle est choisie parmi les poudres de silsesquioxane, le talc, les poudres de polyamide, et leurs mélanges.

37. Composition selon la revendication 35 ou 36, **caractérisée par le fait que** la charge additionnelle est présente en une teneur allant de 0,1 % à 35 % en poids, par rapport au poids total de la composition, de préférence allant de 0,5 % à 30 % en poids, et préférentiellement allant de 1 % à 25 % en poids.

38. Composition selon l'une quelconque des revendications précédentes, **caractérisée par le fait qu'**elle comprend une poudre de silsesquioxane en une teneur allant de 0,5 % à 30 % en poids, par rapport au poids total de la composition, et de préférence allant de 1 % à 15 % en poids, et préférentiellement allant de 1 % à 10 % en poids.

39. Composition selon l'une quelconque des revendications précédentes, **caractérisée par le fait qu'**elle comprend une teneur totale en matières pulvérulentes allant de 20 % à 50 % en poids, par rapport au poids total de la composition, de préférence allant de 25 % à 45 % en poids, et préférentiellement allant de 30 % à 40 % en poids.

40. Composition selon l'une quelconque des revendications précédentes, **caractérisée par le fait qu'**elle comprend un épaississant minéral de milieu huileux.

41. Composition selon la revendication précédente, **caractérisée par le fait que** l'agent épaississant minéral de milieu huileux est choisi parmi les argiles organophiles et les silices pyrogénées.

42. Composition selon l'une quelconque des revendications 40 et 41, **caractérisée par le fait que** l'agent épaississant minéral de milieu huileux est présent en une teneur allant de 0,5 % à 5 % en poids, par rapport au poids total de la composition, de préférence en une teneur allant de 1 % à 4 % en poids, et préférentiellement allant de 2 % à 4 % en poids.

43. Composition selon l'une quelconque des revendications précédentes, **caractérisée par le fait qu'**elle comprend un ingrédient cosmétique choisi parmi les antioxydants, les parfums, les conservateurs, les neutralisants, les tensioactifs, les filtres solaires, les vitamines, les hydratants, les composée auto-bronzants, les actifs antirides.

44. Composition selon l'une quelconque des revendications précédentes, **caractérisée par le fait qu'**elle contient moins de 3 % en poids, par rapport au poids total de la composition de particules de polymère dispersées dans la phase huileuse de la composition et stabilisées en surface par un polymère stabilisant.

45. Composition selon l'une quelconque des revendications précédentes, **caractérisée par le fait qu'**elle est un fond de teint, un fard à paupières, un fard à joue, un produit anticernes, un produit de maquillage du corps, une base de maquillage, un produit matifiant pour la peau, un produit de soin de la peau.

46. Composition selon l'une quelconque des revendications précédentes, **caractérisée par le fait qu'**elle se présente sous la forme d'un gel non fluide déformable.

47. Fond de teint comprenant une composition selon l'une quelconque des revendications 1 à 46.

48. Procédé cosmétique de maquillage ou de traitement non thérapeutique de la peau comprenant l'application sur la peau d'une composition selon l'une quelconque des revendications précédentes.

49. Utilisation d'une composition selon l'une quelconque des revendications 1 à 47 pour obtenir un maquillage ayant une bonne tenue, notamment après 12 heures, et/ou une peau maquillée douce et/ou un maquillage confortable et/ou un maquillage ayant un aspect poudré et/ou un maquillage homogène.

## Claims

1. Anhydrous composition for making up or caring for the skin comprising less than 2% by weight of water and comprising an oily phase, an amorphous gelling polymer formed by polymerization of an olefin and at least one non-film-forming polyurethane powder.

2. Composition according to the preceding claim, **characterized in that** the polymeric gelling agent is an amorphous block copolymer of styrene and of olefin.

3. Composition according to either one of the preceding claims, **characterized in that** the polymeric gelling agent is formed by polymerization of ethylenic carbon monomers having in particular one or two ethylenic unsaturations and having from 2 to 5 carbon atoms.

4. Composition according to any one of the preceding claims, **characterized in that** the polymeric gelling agent is formed by polymerization of an olefin chosen from ethylene, propylene, butadiene or isoprene.

5. Composition according to any one of the preceding claims, **characterized in that** the polymeric gelling agent is chosen from diblock, triblock, multiblock, radial or star copolymers, or their blends.

6. Composition according to any one of the preceding claims, **characterized in that** the polymeric gelling agent is chosen from triblock, multiblock, radial or star copolymers, or their blends.

7. Composition according to any one of the preceding claims, **characterized in that** the polymeric gelling agent is an optionally hydrogenated copolymer comprising styrene blocks and comprising ethylene/C₃-C₄ alkylene blocks.

8. Composition according to any one of Claims 1 to 5 and 7, **characterized in that** the polymeric gelling agent is a preferably hydrogenated diblock copolymer chosen from styrene-ethylene/propylene or styrene-ethylene/butadiene copolymers and preferably a styrene-ethylene/propylene diblock copolymer.

9. Composition according to any one of Claims 1 to 7, **characterized in that** the polymeric gelling agent is a preferably hydrogenated triblock copolymer chosen from styrene-ethylene/propylene-styrene copolymers, styrene-ethylene/butadiene-styrene copolymers, styreneisoprene-styrene copolymers or styrene-butadienestyrene copolymers.

10. Composition according to any one of Claims 1 to 7, **characterized in that** the polymeric gelling agent is a blend of hydrogenated styrene-butylene/ethylene-styrene triblock copolymer and of hydrogenated styrene-propylene-styrene star polymer.

11. Composition according to any one of the preceding claims, **characterized in that** the polymeric gelling agent is present in a content ranging from 0.1% to 10% by weight, with respect to the total weight of the composition, preferably ranging from 0.5% to 5% by weight and preferentially ranging from 1% to 3% by weight.

12. Composition according to any one of the preceding claims, **characterized in that** the polyurethane powder is a powder formed of hexamethylene diisocyanate and trimethylol hexyllactone copolymer.

13. Composition according to any one of the preceding claims, **characterized in that** it comprises a polyurethane powder in a content ranging from 0.5% to 30% by weight, with respect to the total weight of the composition, and preferably ranging from 1% to 15% by weight and preferentially ranging from 5% to 15% by weight.

14. Composition according to any one of the preceding claims, **characterized in that** it comprises poly(methyl methacrylate) particles.

15. Composition according to any one of the preceding claims, **characterized in that** the poly(methyl methacrylate) particles are present in a content ranging from 0.5% to 30% by weight, with respect to the total weight of the composition, preferably ranging from 0.5% to 25% by weight, preferentially ranging from 1% to 20% by weight and more preferentially ranging from 5% to 15% by weight.

16. Composition according to any one of the preceding claims, **characterized in that** it comprises a volatile oil.

17. Composition according to any one of the preceding claims, **characterized in that** it comprises a volatile hydrocarbon oil.

18. Composition according to any one of the preceding claims, **characterized in that** it comprises a volatile hydrocarbon oil chosen from volatile hydrocarbon oils having from 8 to 16 carbon atoms, and their mixtures, and in particular branched C₈-C₁₆ alkanes.

19. Composition according to any one of the preceding claims, **characterized in that** it comprises a volatile hydrocarbon oil chosen from isododecane, isodecane, isohexadecane, and their mixtures.

20. Composition according to any one of the preceding claims, **characterized in that** it comprises a mixture of isododecane and of isohexadecane.

21. Composition according to any one of the preceding claims, **characterized in that** it comprises a volatile silicone oil.

22. Composition according to the preceding claim, **characterized in that** the volatile silicone oil is chosen from octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane and their mixtures.

23. Composition according to any one of Claims 16 to 22, **characterized in that** the volatile oil is present in a content ranging from 5% to 60% by weight, with respect to the total weight of the composition, preferably ranging from 10% to 55% by weight, preferentially ranging from 20% to 50% by weight and more preferentially ranging from 30% to 50% by weight.

24. Composition according to any one of the preceding claims, **characterized in that** it comprises a non-volatile oil.

25. Composition according to any one of the preceding claims, **characterized in that** it comprises a non-volatile oil chosen from non-volatile hydrocarbon oils, non-volatile silicone oils, and their mixtures.

26. Composition according to any one of the preceding claims, **characterized in that** it comprises a non-volatile oil chosen from C₁₂-C₃₆ esters.

27. Composition according to any one of Claims 24 to 26, **characterized in that** the non-volatile oil is present in a content ranging from 0.5% to 60% by weight, with respect to the total weight of the composition, preferably ranging from 1% to 50% by weight, preferably ranging from 5% to 40% by weight, preferentially ranging from 5% to 30% by weight and more preferentially ranging from 10% to 20% by weight.

28. Composition according to any one of Claims 24 to 27, in combination with either of Claims 13 and 14, **characterized in that** the non-volatile oil is present in a content greater than the content of poly(methyl methacrylate) particles.

29. Composition according to any one of Claims 24 to 28, in combination with either of Claims 13 and 14, **characterized in that** the non-volatile oil and the poly(methyl methacrylate) particles are present in a content such that the non-volatile oil/poly(methyl methacrylate) particles ratio by weight is greater than or equal to 1, in particular ranging from 1 to 35, preferably ranging from 1 to 25 and preferentially ranging from 1 to 15.

30. Composition according to any one of Claims 24 to 28, in combination with either of Claims 13 and 14, **characterized in that** the non-volatile oil and the poly(methyl methacrylate) particles are present in a content such that the non-volatile oil/poly(methyl methacrylate) particles ratio by weight is greater than or equal to 1.5, in particular ranging from 1.5 to 35, preferably ranging from 1.5 to 25 and preferentially ranging from 1.5 to 15.

31. Composition according to any one of the preceding claims, **characterized in that** it comprises at least one colouring material.

32. Composition according to the preceding claim, **characterized in that** the colouring material is chosen from pigments, pearlescent agents, fat-soluble dyes, and their mixtures.

33. Composition according to either of Claims 31 and 32, **characterized in that** the colouring material is present in a content ranging from 0.1% to 30% by weight, with respect to the total weight of the composition, preferably ranging from 0.1% to 20% by weight, preferably ranging from 0.5% to 15% by weight, preferentially ranging from 1% to 15% by weight and more preferentially ranging from 5% to 15% by weight.

34. Composition according to any one of the preceding claims, **characterized in that** it comprises an additional filler other than the polyurethane powder and the poly(methyl methacrylate) particles.

35. Composition according to the preceding claim, **characterized in that** the additional filler is chosen from talc, mica, silica, kaolin, polyamide powders, poly-β-alanine powders, polyethylene powders, powders formed of tetrafluoroethylene polymers, lauroyllysine, starch, boron nitride, hollow microspheres formed of poly(vinylidene chloride)/acrylonitrile, hollow microspheres formed of acrylic acid copolymers, silicone resin powders, polyorganosiloxane elastomer particles, precipitated calcium carbonate, magnesium carbonate, basic magnesium carbonate, hydroxyapatite, glass or ceramic microcapsules, metal soaps derived from organic carboxylic acids having from 8 to 22 carbon atoms, and their mixtures.

36. Composition according to the preceding claim, **characterized in that** the additional filler is chosen from silsesquioxane powders, talc, polyamide powders, and their mixtures.

37. Composition according to Claim 35 or 36, **characterized in that** the additional filler is present in a content ranging from 0.1% to 35% by weight, with respect to the total weight of the composition, preferably ranging from 0.5% to 30% by weight and preferentially ranging from 1% to 25% by weight.

38. Composition according to any one of the preceding claims, **characterized in that** it comprises a silsesquioxane powder in a content ranging from 0.5% to 30% by weight, with respect to the total weight of the composition, and preferably ranging from 1% to 15% by weight and preferentially ranging from 1% to 10% by weight.

39. Composition according to any one of the preceding claims, **characterized in that** it comprises a total content of pulverulent materials ranging from 20% to 50% by weight, with respect to the total weight of the composition, preferably ranging from 25% to 45% by weight and preferentially ranging from 30% to 40% by weight.

40. Composition according to any one of the preceding claims, **characterized in that** it comprises an inorganic thickener for an oily medium.

41. Composition according to the preceding claim, **characterized in that** the inorganic thickener for an oily medium is chosen from organophilic clays and pyrogenic silicas.

42. Composition according to either one of Claims 40 and 41, **characterized in that** the inorganic thickener for an oily medium is present in a content ranging from 0.5% to 5% by weight, with respect to the total weight of the composition, preferably in a content ranging from 1% to 4% by weight and preferentially ranging from 2% to 4% by weight.

43. Composition according to any one of the preceding claims, **characterized in that** it comprises a cosmetic ingredient chosen from antioxidants, fragrances, preservatives, neutralizing agents, surfactants, sunscreen agents, vitamins, moisturizing agents, self-tanning compounds or antiwrinkle active principles.

44. Composition according to any one of the preceding claims, **characterized in that** it comprises less than 3% by weight, with respect to the total weight of the composition, of polymer particles dispersed in the oily phase of the composition and stabilized at the surface by a stabilizing polymer.

45. Composition according to any one of the preceding claims, **characterized in that** it is a foundation, an eye shadow, a blusher, a concealer, a product for making up the body, a make-up base, a mattifying product for the skin or a product for caring for the skin.

46. Composition according to any one of the preceding claims, **characterized in that** it is provided in the form of a deformable non-fluid gel.

47. Foundation comprising a composition according to any one of Claims 1 to 46.

48. Cosmetic process for making up or for the non-therapeutic treatment of the skin, comprising the application, to the skin, of a composition according to any one of the preceding claims.

49. Use of a composition according to any one of Claims 1 to 47 to produce a make-up having good hold, in particular after 12 hours, and/or a soft made-up skin and/or a comfortable make-up and/or a make-up having a powdery appearance and/or a homogeneous make-up.

## Patentansprüche

1. Wasserfreie Zusammensetzung zum Schminken oder zur Pflege der Haut, die weniger als 2 Gew.-% Wasser enthält und eine Ölphase, ein durch Polymerisation eines Olefins erzeugtes amorphes polymeres Gelbildungsmittel und mindestens ein Pulver aus einem nicht-filmbildenden Polyurethan aufweist.

2. Zusammensetzung nach dem vorhergehenden Anspruch, **dadurch gekennzeichnet, dass** das polymere Gelbildungsmittel ein amorphes Blockcopolymer von Styrol und einem Olefin ist.

3. Zusammensetzung nach einem der vorhergehenden Ansprüche, **dadurch gekennzeichnet, dass** das polymere Gelbildungsmittel durch Polymerisation von ethylenisch ungesättigten Monomeren, insbesondere mit einer oder zwei ethylenisch ungesättigten Bindungen, mit 2 bis 5 Kohlenstoffatomen erzeugt ist.

4. Zusammensetzung nach einem der vorhergehenden Ansprüche, **dadurch gekennzeichnet, dass** das polymere Gelbildungsmittel durch Polymerisation eines Olefins erzeugt ist, das unter Ethylen, Propylen, Butadien und Isopren ausgewählt ist.

5. Zusammensetzung nach einem der vorhergehenden Ansprüche, **dadurch gekennzeichnet, dass** das polymere Gelbildungsmittel unter Diblockcopolymeren, Triblockcopolymeren, Multiblockcopolymeren, radialen Copolymeren, Sterncopolymeren, oder ihren Gemischen ausgewählt ist.

6. Zusammensetzung nach einem der vorhergehenden Ansprüche, **dadurch gekennzeichnet, dass** das polymere Gelbildungsmittel unter Triblockcopolymeren, Multiblockcopolymeren, radialen Copolymeren, Sterncopolymeren oder ihren Gemischen ausgewählt ist.

7. Zusammensetzung nach einem der vorhergehenden Ansprüche, **dadurch gekennzeichnet, dass** das polymere Gelbildungsmittel ein gegebenenfalls hydriertes Copolymer mit Styrolblöcken und Ethylen/ C₃-C₄-Alkylen-Blöcken ist.

8. Zusammensetzung nach einem der Ansprüche 1 bis 5 und 7, **dadurch gekennzeichnet, dass** das polymere Gelbildungsmittel ein Diblockcopolymer ist, das vorzugsweise hydriert ist und das unter Styrol-Ethylen/Propylen-Copolymeren, Styrol-Ethylen/Butadien-Copolymeren ausgewählt ist und vorzugweise ein Styrol-Ethylen/Propylen-Diblockcopolymer ist.

9. Zusammensetzung nach einem der Ansprüche 1 bis 7, **dadurch gekennzeichnet, dass** das polymere Gelbildungsmittel ein Triblockcopolymer ist, das vorzugweise hydriert ist und das ausgewählt ist unter Styrol-Ethylen/Propylen-Styrol-Copolymeren, Styrol-Ethylen/Butadien-Styrol-Copolymeren, Styrol-Isopren-Styrol-Copolymeren und Styrol-Butadien-Styrol-Copolymeren.

10. Zusammensetzung nach einem der Ansprüche 1 bis 7, **dadurch gekennzeichnet, dass** das polymere Gelbildungsmittel ein Gemisch eines hydrierten Styrol-Butylen/Ethylen-Styrol-Copolymers und eines hydrierten Styrol-Propylen-Styrol-Sternpolymers ist.

11. Zusammensetzung nach einem der vorhergehenden Ansprüche, **dadurch gekennzeichnet, dass** das polymere Gelbildungsmittel in einem Mengenanteil von 0,1 bis 10 Gew.-%, bevorzugt in einem Mengenanteil von 0,5 bis 5 Gew.-% und noch bevorzugter in einem Mengenanteil von 1 bis 3 Gew.-% vorliegt, bezogen auf das Gesamtgewicht der Zusammensetzung.

12. Zusammensetzung nach einem der vorhergehenden Ansprüche, **dadurch gekennzeichnet, dass** das Polyurethanpulver ein Pulver aus einem Copolymer aus Hexamethylendiisocyanat und Trimethylolhexyllacton ist.

13. Zusammensetzung nach einem der vorhergehenden Ansprüche, **dadurch gekennzeichnet, dass** sie ein Polyurethanpulver in einem Mengenanteil von 0,5 bis 30 Gew.-%, bevorzugt in einem Mengenanteil von 1 bis 15 Gew.-% und noch bevorzugter in einem Mengenanteil von 5 bis 15 Gew.-% enthält, bezogen auf das Gesamtgewicht der Zusammensetzung.

14. Zusammensetzung nach einem der vorhergehenden Ansprüche, **dadurch gekennzeichnet, dass** sie Partikel aus Polymethylmethacrylat enthält.

15. Zusammensetzung nach einem der vorhergehenden Ansprüche, **dadurch gekennzeichnet, dass** die Partikel aus Polymethylmethacrylat in einem Mengenanteil von 0,5 bis 30 Gew.-%, vorzugweise in einem Mengenanteil von 0,5 bis 25 Gew.-%, bevorzugt in einem Mengenanteil von 1 bis 20 Gew.-% und noch bevorzugter in einem Mengenanteil von 5 bis 15 Gew.-% vorliegen, bezogen auf das Gesamtgewicht der Zusammensetzung.

16. Zusammensetzung nach einem der vorhergehenden Ansprüche, **dadurch gekennzeichnet, dass** sie ein flüchtiges Öl enthält.

17. Zusammensetzung nach einem der vorhergehenden Ansprüche, **dadurch gekennzeichnet, dass** sie ein flüchtiges Kohlenwasserstofföl enthält.

18. Zusammensetzung nach einem der vorhergehenden Ansprüche, **dadurch gekennzeichnet, dass** sie ein flüchtiges Kohlenwasserstofföl enthält, das unter flüchtigen Kohlenwasserstoffölen mit 8 bis 16 Kohlenstoffatomen und ihren Gemischen und insbesondere verzweigten C₈-C₁₆-Alkanen ausgewählt ist.

19. Zusammensetzung nach einem der vorhergehenden Ansprüche, **dadurch gekennzeichnet, dass** sie ein flüchtiges Kohlenwasserstofföl enthält, das unter Isododecan, Isodecan und Isohexadecan sowie ihren Gemischen ausgewählt ist.

20. Zusammensetzung nach einem der vorhergehenden Ansprüche, **dadurch gekennzeichnet, dass** sie ein Gemisch von Isododecan und Isohexadecan enthält.

21. Zusammensetzung nach einem der vorhergehenden Ansprüche, **dadurch gekennzeichnet, dass** sie ein flüchtiges Siliconöl enthält.

22. Zusammensetzung nach dem vorhergehenden Anspruch, **dadurch gekennzeichnet, dass** das flüchtige Siliconöl ausgewählt ist unter Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan und Dodecamethylpentasiloxan sowie ihren Gemischen.

23. Zusammensetzung nach einem der Ansprüche 16 bis 22, **dadurch gekennzeichnet, dass** das flüchtige Öl in einem Mengenanteil von 5 bis 60 Gew.-%, vorzugweise in einem Mengenanteil von 10 bis 55 Gew.-%, bevorzugt in einem Mengenanteil von 20 bis 50 Gew.-% und noch bevorzugter in einem Mengenanteil von 30 bis 50 Gew.-% vorliegt, bezogen auf das Gesamtgewicht der Zusammensetzung.

24. Zusammensetzung nach einem der vorhergehenden Ansprüche, **dadurch gekennzeichnet, dass** sie ein nichtflüchtiges Öl enthält.

25. Zusammensetzung nach einem der vorhergehenden Ansprüche, **dadurch gekennzeichnet, dass** sie ein nichtflüchtiges Öl enthält, das unter nichtflüchtigen Kohlenwasserstoffölen und nichtflüchtigen Siliconölen sowie ihren Gemischen ausgewählt ist.

26. Zusammensetzung nach einem der vorhergehenden Ansprüche, **dadurch gekennzeichnet, dass** sie ein nichtflüchtiges Öl enthält, das unter den C₁₂-C₃₆-Estern ausgewählt ist.

27. Zusammensetzung nach einem der Ansprüche 24 bis 26, **dadurch gekennzeichnet, dass** das nichtflüchtige Öl in einem Mengenanteil von 0,5 bis 60 Gew.-%, vorzugweise in einem Mengenanteil von 1 bis 50 Gew.-%, bevorzugt in einem Mengenanteil von 5 bis 40 Gew.-%, noch bevorzugter in einem Mengenanteil von 5 bis 30 Gew.-% und besonders bevorzugt in einem Mengenanteil von 10 bis 20 Gew.-% vorliegt, bezogen auf das Gesamtgewicht der Zusammensetzung.

28. Zusammensetzung nach einem der Ansprüche 24 bis 27 in Kombination mit einem der Ansprüche 13 und 14, **dadurch gekennzeichnet, dass** das nichtflüchtige Öl in einem Mengenanteil vorliegt, der größer ist als der Mengenanteil der Partikel aus Polymethylmethacrylat.

29. Zusammensetzung nach einem der Ansprüche 24 bis 28 in Kombination mit einem der Ansprüche 13 und 14, **dadurch gekennzeichnet, dass** das nichtflüchtige Öl und die Partikel aus Polymethylmethacrylat in einem solchen Mengenanteil vorliegen, dass das Gewichtsverhältnis nichtflüchtiges Öl/Partikel aus Polymethylmethacrylat größer als oder gleich 2 ist und insbesondere im Bereich von 1 bis 35, vorzugweise im Bereich von 1 bis 25 und bevorzugt im Bereich von 1 bis 15 liegt.

30. Zusammensetzung nach einem der Ansprüche 24 bis 28 in Kombination mit einem der Ansprüche 13 und 14, **dadurch gekennzeichnet, dass** das nichtflüchtige Öl und die Partikel aus Polymethylmethacrylat in einem solchen Mengenanteil vorliegen, dass das Gewichtsverhältnis nichtflüchtiges Öl/Partikel aus Polymethylmethacrylat größer als oder gleich 1,5 ist und insbesondere im Bereich von 1,5 bis 35, vorzugweise im Bereich von 1,5 bis 25 und bevorzugt im Bereich von 1,5 bis 15 liegt.

31. Zusammensetzung nach einem der vorhergehenden Ansprüche, **dadurch gekennzeichnet, dass** sie mindestens ein Färbemittel enthält.

32. Zusammensetzung nach dem vorhergehenden Anspruch, **dadurch gekennzeichnet, dass** das Färbemittel unter Pigmenten, Perlglanzmitteln und öllöslichen Farbstoffen sowie ihren Gemischen ausgewählt ist.

33. Zusammensetzung nach Anspruch 31 oder 32, **dadurch gekennzeichnet, dass** das Färbemittel in einem Mengenanteil von 0,1 bis 30 Gew.-%, vorzugweise in einem Mengenanteil von 0,1 bis 20 Gew.-%, bevorzugt in einem Mengenanteil von 0,5 bis 15 Gew.-%, noch bevorzugter in einem Mengenanteil von 1 bis 15 Gew.-% und besonders bevorzugt in einem Mengenanteil von 5 bis 15 Gew.-% vorliegt, bezogen auf das Gesamtgewicht der Zusammensetzung.

34. Zusammensetzung nach einem der vorhergehenden Ansprüche, **dadurch gekennzeichnet, dass** sie einen weiteren Füllstoff enthält, der von dem Polyurethanpulver und den Partikeln aus Polymethylmethacrylat verschieden ist.

35. Zusammensetzung nach dem vorhergehenden Anspruch, **dadurch gekennzeichnet, dass** der zusätzliche Füllstoff ausgewählt ist unter Talk, Glimmer, Kieselsäure, Kaolin, Pulvern aus Polyamid, Pulvern aus Poly-β-alanin, Pulvern aus Polyethylen, Pulvern aus Tetrafluoroethylen-Polymeren, Lauroyllysin, Stärke, Bornitrid, hohlen Mikrokügelchen aus Polyvinylidenchlorid/Acrylnitril, hohlen Mikrokügelchen aus Acrylsäure-Copolymeren, Pulvern aus Siliconharz, Partikeln aus Polyorganosiloxan-Elastomeren, gefälltem Calciumcarbonat, Magnesiumcarbonat, Magnesiumhydrogencarbonat, Hydroxylapatit, Mikrokapseln aus Glas oder Keramik und von organischen Carbonsäuren mit 8 bis 22 Kohlenstoffatomen abgeleiteten Metallseifen sowie Gemischen dieser Stoffe.

36. Zusammensetzung nach dem vorhergehenden Anspruch, **dadurch gekennzeichnet, dass** der zusätzliche Füllstoff unter Pulvern aus Silsesquioxan, Talk und Pulvern aus Polyamid sowie Gemischen dieser Stoffe ausgewählt ist.

37. Zusammensetzung nach Anspruch 35 oder 36, **dadurch gekennzeichnet, dass** der zusätzliche Füllstoff in einem Mengenanteil von 0,1 bis 35 Gew.-%, vorzugweise in einem Mengenanteil von 0,5 bis 30 % Gew.-% und bevorzugt in einem Mengenanteil von 1 bis 25 Gew.-% vorliegt, bezogen auf das Gesamtgewicht der Zusammensetzung.

38. Zusammensetzung nach einem der vorhergehenden Ansprüche, **dadurch gekennzeichnet, dass** sie ein Pulver aus Silsesquioxan in einem Mengenanteil von 0,5 bis 30 Gew.-%, vorzugweise in einem Mengenanteil von 1 bis 15 Gew.-% und bevorzugt in einem Mengenanteil von 1 bis 10 Gew.-% enthält, bezogen auf das Gesamtgewicht der Zusammensetzung.

39. Zusammensetzung nach einem der vorhergehenden Ansprüche, **dadurch gekennzeichnet, dass** sie einen Gesamtgehalt an pulverförmigen Stoffen von 20 bis 50 Gew.-%, vorzugweise von 25 bis 45 Gew.-% und bevorzugt 30 bis 40 Gew.-% aufweist, bezogen auf das Gesamtgewicht der Zusammensetzung.

40. Zusammensetzung nach einem der vorhergehenden Ansprüche, **dadurch gekennzeichnet, dass** sie ein anorganisches Verdickungsmittel für das Ölmedium enthält.

41. Zusammensetzung nach dem vorhergehenden Anspruch, **dadurch gekennzeichnet, dass** das anorganische Verdickungsmittel für das Ölmedium unter organophilen Tonen und pyrogenen Kieselsäuren ausgewählt ist.

42. Zusammensetzung nach einem der Ansprüche 40 und 41, **dadurch gekennzeichnet, dass** das anorganische Verdickungsmittel für das Ölmedium in einem Mengenanteil von 0,5 bis 5 Gew.-%, vorzugweise in einem Mengenanteil von 1 bis 4 Gew.-% und bevorzugt in einem Mengenanteil von 2 bis 4 Gew.-% vorliegt, bezogen auf das Gesamtgewicht der Zusammensetzung.

43. Zusammensetzung nach einem der vorhergehenden Ansprüche, **dadurch gekennzeichnet, dass** sie einen kosmetischen Bestandteil enthält, der ausgewählt ist unter Antioxidationsmitteln, Parfums, Konservierungsmitteln, Neutralisierungsmitteln, Tensiden, Sonnenfiltern, Vitaminen, Hydratationsmitteln, selbstbräunenden Verbindungen und Wirkstoffen gegen Falten.

44. Zusammensetzung nach einem der vorhergehenden Ansprüche, **dadurch gekennzeichnet, dass** sie weniger als 3 Gew.-%, bezogen auf das Gesamtgewicht der Zusammensetzung, an Polymerpartikeln enthält, die in der Ölphase der Zusammensetzung dispergiert und auf ihrer Oberfläche durch ein stabilisierendes Polymer stabilisiert sind.

45. Zusammensetzung nach einem der vorhergehenden Ansprüche, **dadurch gekennzeichnet, dass** es sich um ein Make-up, einen Lidschatten, ein Rouge, ein Produkt gegen Falten, ein Produkt zum Schminken des Körpers, eine Schminkgrundlage, ein Produkt zur Mattierung der Haut oder ein Produkt zur Pflege der Haut handelt.

46. Zusammensetzung nach einem der vorhergehenden Ansprüche, **dadurch gekennzeichnet, dass** sie in Form eines verformbaren, nicht-flüssigen Gels vorliegt.

47. Make-up, das eine Zusammensetzung nach einem der Ansprüche 1 bis 46 enthält.

48. Kosmetisches Verfahren zum Schminken oder zur nichttherapeutischen Behandlung der Haut, das die Anwendung einer Zusammensetzung nach einem der vorhergehenden Ansprüche auf die Haut umfasst.

49. Verwendung einer Zusammensetzung nach einem der Ansprüche 1 bis 47 zur Erzielung einer Schminke mit guter Haltbarkeit, insbesondere nach 12 Stunden, und/oder einer milden geschminkten Haut und/oder einer angenehmen Schminke und/oder einer Schminke mit gepudertem Aussehen und/oder einer gleichmäßigen Schminke.
